# FASCICULE DE BREVET EUROPEEN

(11) **EP 2 343 038 B1**
(45) Date de publication et mention de la délivrance du brevet: **26.11.2014**
(21) Numéro de dépôt: 10195981.5
(22) Date de dépôt: 20.12.2010
(51) Int. Cl.: A61K 8/27, A61K 8/37, A61K 9/06, A61K 31/25, A61K 33/30, A61Q 19/00, A61K 8/92, A61K 8/96, A61P 31/22

(54) **Compositions dermatologiques contenant une association de lipides peroxydés et de zinc et leurs utilisations notamment dans le traitement de l'herpès**
Dermatologische Zubereitugen enthaltend eine Kombination von peroxydierten Fetten und Zink und Verwendung davon in der Behandlung von unter anderem Herpes.
Dermatological compositions comprising a combination of peroxidized lipids and zinc for use amongst others in the treatment of herpes.

(30) Priorité: 24.12.2009 FR 0959604
(43) Date de publication de la demande: 13.07.2011
(73) Titulaire: Laboratoires Carilene, 78360 Montesson (FR); Desjonqueres, Stéphane, 78600 Maisons Laffitte (FR)
(72) Inventeur: Desjonqueres, Stéphane, 78600, MAISONS LAFFITTE (FR)
(74) Mandataire: Hubert, Philippe

(56) Documents cités:
- EP-A2- 0 225 831
- WO-A1-97/26892
- WO-A1-2009/060493
- CA-A1- 2 514 381
- DE-A1- 10 161 729
- FR-A1- 2 461 744
- FR-A1- 2 824 474
- US-B1- 6 686 392
- DATABASE WPI Week 198733 Thomson Scientific, London, GB; AN 1987-230746 XP002587043, HATA A; MATSUMIYA T: "Cataplasm for external application - includes base contg. vinyl! chloride-ethylene! copolymer, ethylene! carbon mon: oxide vinyl! acetate copolymer and aluminosilicate", -& JP 62 153215 A (SEKISUI CHEM IND CO LTD) 8 juillet 1987 (1987-07-08)
- KNEIST W ET AL: "KLINISCHE DOPPELBLIND PRUEFUNG MIT ZINKSULFAT TOPISCH BEI HERPES LABIALIS RECIDIVANS", ARZNEIMITTEL FORSCHUNG. DRUG RESEARCH, ECV EDITIO CANTOR VERLAG, AULENDORF, DE, vol. 45 (I), no. 5, 1 janvier 1995 (1995-01-01), pages 624-626, XP008065510, ISSN: 0004-4172

## Description

La présente invention concerne de nouvelles compositions dermatologiques contenant une association de lipides peroxydés et de zinc et leurs utilisations notamment dans le traitement de l'herpès.

### ETAT DE LA TECHNIQUE

L'herpès est une affection très fréquente dont les manifestations se résument habituellement à des signes cutanéo-muqueux avec leur séquence particulière: primo- infection, latence, récurrences multiples. Les lésions d'herpès sont spontanément résolutives mais elles se caractérisent par un préjudice esthétique et des signes fonctionnels générateurs d'inconfort. Le responsable est le virus HSV (Herpès Virus Simplex). C'est un virus à ADN de 150 à 2000 nm. Deux types antigéniques peuvent être distingués: le HSV1 qui infecte plutôt la partie supérieure du corps (le principal responsable de l'herpès labial) et le HSV2 responsable de l'herpès de la région génitale.

L'herpès génital est une maladie sexuellement transmissible fréquente. Elle toucherait 2 millions de personnes en France. Ses symptômes sont pénibles et récidivants et peuvent perturber la vie sexuelle des patients. La primo-infection à virus Herpès simplex débute 7 à 21 jours après le contact sexuel infectant. Très spectaculaire, elle se manifeste:
- Chez l'homme par une balano-posthite avec urétrite ;
- Chez la femme par une vulvo-vaginite aiguë fébrile avec méatite ou cervicite (atteinte du méat urinaire ou du col/utérin).

L'herpès génital est généralement dû au virus Herpes Simplex de type 2 (HSV2), mais HSV 1 (impliqué dans l'herpès labial) peut également être en cause. Il existe *également* de nombreuses formes asymptomatiques d'herpès génital (la moitié des patients infectés ne présentent aucun signe).

La crise d'herpès suit plusieurs étapes : à l'endroit de l'éruption cutanée, le patient ressent des démangeaisons et des brûlures fugaces ; 24 à 48 heures plus tard, de petites vésicules apparaissent, groupées en bouquets sur les régions atteintes. Des signes généraux peuvent être présents (fièvre, malaise). Quelques jours après, les vésicules éclatent pour laisser place à de petites érosions suintantes douloureuses ; c'est ensuite l'apparition de croûtes qui signent la fin de la crise qui aura duré de 4 à 8 jours.

Les muqueuses atteintes chez la femme sont la vulve, la région anale et l'entrée vaginale ; chez l'homme, le gland, le fourreau, le sillon balano-préputial et la région anale. La lésion herpétique est douloureuse, ne repose pas sur une base indurée et s'accompagne d'adénopathies. Elle est contagieuse pendant 2 semaines.

Après une première crise d'herpès génital, 50 à 70 % des patients ne souffriront plus de manifestations herpétiques. Les autres présenteront des récidives fréquentes lors de facteurs déclenchant : stress, menstruations, grippe, corticothérapie ... Au moment de la crise, le virus resté latent dans les ganglions lymphatiques rejoint la peau en suivant les nerfs et provoque les lésions. Les récidives se présentent sous forme de vésicules sur le pénis, la vulve, le col de l'utérus, mais parfois également sur les fesses ou les jambes. Elles sont annoncées par une douleur locale que le patient apprend à reconnaître. Plus l'infection primaire a été sévère, plus les patients ont tendance à présenter de récidives. A long terme, les récidives tendent à devenir moins fréquentes.

Il existe des traitements qui traitent la crise mais qui ne permettent pas de supprimer le virus, qui reste à l'état latent dans les ganglions lymphatiques entre les poussées. Ces traitements reposent principalement sur l'utilisation de substances antivirales par voie locale (crèmes ou gels) ou orale (comprimés ou solutions buvables).

L'objectif du traitement de la poussée d'herpès labial aussi bien génital est la diminution des signes fonctionnels, du délai de guérison et dans la durée, de la contagiosité. Les traitements antiviraux locaux généralement utilisés (crèmes à base d'acyclovir 5 %, telles que le ZOVIRAX®) peuvent accélérer la guérison mais n'agissent pas sur les signes fonctionnels qui peuvent être très invalidants.

Il a été décrit dans la demande précédente du Déposant FR 2.591.109 une composition médicamenteuse à base d'huiles naturelles d'origine végétale hyperoxygénées comprenant des TriGlycérides Peroxydés ou en abrégé TGO, pour le traitement de l'Herpès. Cette huile est par exemple choisie parmi l'huile d'arachide, l'huile d'amande douce et l'huile de carthame , voir notamment les revendications 1 et 2. Cependant cette composition a ultérieurement été jugée comme non significativement efficace pour le traitement de l'Herpès.

Le zinc et un certain nombre de ses composés tels que l'oxyde de zinc et également l'acétate, le carbonate, le sulfate, le phosphate, le chlorure, le distéarate, le gluconate, sont bien connus pour leurs propriétés antiseptiques ou antifongiques et pour leurs actions contre les démangeaisons.

Ainsi, des pommades ou lotions à l'oxyde de zinc ont été, de longues dates, utilisées contre les démangeaisons ou pour traiter par exemple l'érythème fessier des bébés. L'oxyde de zinc est également connu pour ses propriétés légèrement antifongiques et s'oppose par conséquent au développement de mycoses.

Il est également connu que l'oxyde de zinc présente une large gamme d'utilisations dont certaines dans le domaine de la cosmétique, notamment pour ses propriétés d'écrans solaires.

On connaît un certain nombre d'onguents ou de lotions renfermant de l'oxyde de zinc, la présence de cet oxyde de zinc prévenant en particulier des irritations de la peau et aidant à rétablir un bon état de la peau.

On connaît également des compositions réparatrices de la peau présentant en outre des propriétés antibactériennes et renfermant de l'oxyde de zinc, ces compositions pouvant être très largement utilisées, notamment chez les enfants atteints d'eczéma ou de croûte de lait.

Le document US 6,886,392 réalise une réaction chimique d'une huile insaturée avec un composé de zinc à une température comprise entre 260 et 280°C pendant environ 15 min à 2 heures pour réaliser une réaction exothermique conduisant à un nouveau produit chimique constitué d'un dérivé de zinc avec de l'huile.

Dans le cas de la présente invention décrite ci-après, aucune réaction chimique n'est réalisée, mais un simple mélange physique des deux composants.

D'autre part, D1 vise l'utilisation de ce composé de zinc spécifiquement pour le traitement de l'hypertrophie de la prostate, ce qui n'a rien à voir avec le traitement de l'herpès, même si ce document cite l'emploi topique comme crème ou lotion pour traiter des dermatoses ou comme agent anti-vieillissement.

D'autre part, le document JP de SEKISUI DATABASE WPI abrégé XP002587043 cite une demande japonaise de Sekisui JP-62/153215 qui vise essentiellement un cataplasme pour utilisation externe dont la partie de base du cataplasme a une composition spécifique (voir les revendications 1 à 7).

La revendication 7 de SEKISUI prévoit que la base de support présente une couche adhésive qui contient un médicament sur une face de celle-ci.

Ce médicament peut être présent dans le cataplasme (page 8, à partir du deuxième paragraphe), et, dans la composition du cataplasme, il peut être utilisé de l'huile de soja époxydée (10 parties en poids), du stéarate de zinc (0,05 partie en poids) parmi les différents ingrédients (voir exemple 1, page 9, lignes 6 et 7 de cet exemple).

L'exemple comparatif contient la même composition si ce n'est l'absence de zéolite A.

Ainsi, les propriétés sont attribuées à la présence ou à l'absence de zéolites.

L'exemple 2, quant à lui ne prévoit pas la présence du stéarate de zinc. Le test effectué concerne uniquement le changement de couleur du médicament, c'est-à-dire sa stabilité.

D'autre part, secondairement, ce document cite la proportion de stéarate de zinc à 0,05 % en poids alors que selon l'invention décrite ci-après il est prévu que le zinc est compris entre 0,1 et 25 % en poids de la composition.

Le document WO 2009/064,193 de Carratelli vise des compositions pharmaceutiques comprenant des huiles peroxydées et du cholestérol et leur utilisation dans le domaine médical pour le traitement des tumeurs, des maladies virales, des ulcères et des dermatites (voir le titre et le premier paragraphe de la page 1).

L'exemple 2, page 5 vise comme traitement de maladie virale le traitement de l'herpès Simplex notamment sur les fesses.

L'activité est attribuée à la combinaison de l'huile peroxydée avec le cholestérol.

Incidemment, l'exemple 4 traite également le traitement des ulcères ou hémorroïdes.

Par ailleurs, la proportion de cholestérol est de 10 % (page 4, lignes 21).

Le Document KNEIST Arzneim.-Forschung./Drug Res. 45 (I), Nr 5 (1995, pages 624-626) présente le résultat d'essais cliniques en double aveugle avec l'emploi du sulfate de zinc appliqué topiquement sous forme de d'un gel de 1 g contenant 10 mg de sulfate de zinc pour le traitement de l'herpès, vis-à-vis d'un placebo.

Ce document conclut à l'efficacité significative de la forme gel du sulfate de zinc pour lutter contre l'herpès labialis recidivans.

Le document EP 0 225 831 Desjonqueres est une invention antérieure du Déposant qui décrit une composition médicamenteuse destinée à être utilisée pour le traitement de l'herpès et qui constitue la composition de comparaison de la description de la présente invention ci-après.

Enfin, le document CA 2514381 est relatif à une préparation topique améliorée pour détruire les cellules infectées par l'Herpes simplex.

Ce brevet utilise une émulsion stable comprenant la combinaison de 30 % en volume à approximativement 50 % en volume d'éther d'origine végétale dans une huile végétale dépourvue d'acides gras libres et essentiellement dépourvue d'eau, de savons et de stabilisants, ainsi que du diméthylsulfoxyde en une proportion de 10 % en volume à environ 20 % en volume; ainsi que 2 % à 8 % en volume de peroxyde de sodium et encore de 2 % à 8 % en volume d'un détergent facilitant la destruction de la matière grasse pour débloquer les cellules du virus pour permettre à l'éther de tuer les cellules.

Ce document, datant de 2005, va dans une direction complètement opposée à l'invention décrite ci-après.

### BUTS DE L'INVENTION

Un but principal de l'invention est de résoudre le problème technique consistant en la fourniture d'une nouvelle composition utilisable par application locale comme médicament de traitement de l'herpès de manière plus efficace que les compositions topiques antérieurement connues.

Un autre but principal de l'invention est de résoudre le problème technique consistant en la fourniture d'une nouvelle composition capable de réaliser le traitement de l'herpès plus efficace non seulement vis-à-vis d'un placébo mais surtout vis-à-vis d'un témoin positif tel que l'agent anti-viral acyclovir (Zovirax^{®})mondialement connu ou que les lipides peroxydés utilisés seuls.

Un autre but principal de l'invention est de résoudre le problème technique consistant en la fourniture d'une nouvelle composition capable de traiter à la fois l'herpès labial et l'herpès génital.

Un autre but principal de la présente invention est de résoudre le problème technique consistant en la fourniture d'une nouvelle composition capable de traiter rapidement et efficacement la douleur associée à l'herpès labial ou génital.

La présente invention a également pour but principal de résoudre le problème technique consistant en la fourniture d'une nouvelle composition capable non seulement de traiter la douleur mais également les critères secondaires de la douleur comprenant la brûlure, le picotement, le prurit ou démangeaison, l'érythème ou encore les signes cliniques accompagnant l'épisode herpétique, principalement oedème, ulcération, érosion, papules, vésicules, croûtes.

De manière plus générale, la présente invention a pour but principal de fournir une nouvelle formulation pharmaceutique pour aider à la cicatrisation ou la régénération de la peau ou des muqueuses, et/ou le renouvellement des cellules du derme et ainsi aider à la reconstitution d'un épiderme lésé et/ou à agir comme agent antalgique sur la peau ou les muqueuses. L'invention a aussi pour but de permettre un traitement de la peau pour des indications de même nature que l'herpès et comprenant le prurit, les brûlures notamment post-traitement de radio-thérapie, les brûlures U.V, en particulier induites par l'exposition au soleil, les démangeaisons et les irritations de grattage.

L'invention a enfin pour but principal de résoudre l'ensemble de ces problèmes techniques selon une nouvelle formulation simple, sûre et fiable, stable, ayant une efficacité répétitive, utilisable à l'échelle industrielle et médicale.

### RESUME DE L'INVENTION

Les essais réalisés par l'inventeur, dans un premier temps dans le cas de l'herpès, ont permis de mettre en évidence un effet de synergie dû à l'addition d'oxyde de zinc comme composé de zinc compatible avec une utilisation dermatologique à une composition topique comprenant des lipides peroxydés ou Triesters de Glycérol Oxydés ou encore en abrégé TGO, résultant en une activité significativement plus efficace contre l'Herpès, notamment par rapport non seulement vis-à-vis d'un placébo mais surtout vis-à-vis d'un témoin positif constitué par le médicament comprenant l'acyclovir à 5% (Zovirax^{®}) mondialement connu ou par rapport aux lipides peroxydés utilisés seuls.

En outre, il a été découvert de manière inattendue que cette association de lipides peroxydés ou TGO ; et d'oxyde de Zinc permettait de rendre la préparation plus pâteuse et adhérente, permettant ainsi d'améliorer grandement l'adhésion du film protecteur formé par les triesters de glycérol oxydés ainsi que l'imprégnation des boutons, conduisant à une diminution de l'apparition des signes fonctionnels et à une accélération de la reconstruction physique du tissu cutané ou muqueux lésé.

Par ailleurs, la lésion d'herpès labial aussi bien que génital comprend deux composantes principales au cours de son développement :
- une composante lésionnelle passant par le stade papule, vésicule, voir bulles avec présence d'érythèmes et de croûtes dans la phase finale ;
- une composante de signes fonctionnels douleurs, brûlures, qui peuvent être très invalidants pour les sujets.

Il est apparu que le soulagement de ces signes fonctionnels pouvait être obtenu aussi bien dans le cas de l'herpès labial que génital, par une application sur les parties de la peau ou des muqueuses atteintes de compositions comprenant l'association de lipides peroxydés et de composé du zinc constitué par de l'oxyde de zinc, qui font l'objet de la présente invention.

Par ailleurs, un autre avantage de la présence d'oxyde de zinc dans la composition est qu'il participe à une meilleure conservation de la composition, du fait des propriétés antibactériennes bien connues de l'oxyde de zinc.

### DESCRIPTION DETAILLEE DE L'INVENTION

### DEFINITIONS

HERPES : l'herpès est une maladie d'origine virale qui s'attaque soit à la peau, soit aux muqueuses de la face, en particulier des lèvres dit herpès labial, soit encore aux muqueuses génitales dit herpès génital, et présente un facteur de récurrence quasi systématique. Le facteur déclenchant des crises d'herpès est souvent d'origine émotive mais peut être aussi d'origine physiologique comme la menstruation et avoir encore d'autres causes extérieures comme l'exposition au soleil, dit herpès solaire, etc. L'herpès se caractérise par la présence de pustules douloureuses, ouvertes et suintantes, la plupart du temps accompagnées d'un caractère inflammatoire très marqué.
HERPES LABIAL : on entend dans la présente description et les revendications par les termes "herpès labial", un herpès localisé sur la face en particulier aux muqueuses de la face et notamment les lèvres. HERPES GENITAL : on entend dans la description et dans les revendications par les termes "herpes génital", un herpès qui se localise sur la peau en dessous du ventre et qui peut aussi inclure les fesses et les muqueuses génitales.
Composition dermatologique : on entend dans la description et dans les revendications par les termes "composition dermatologique", une composition destinée à être appliquée topiquement sur la peau, le derme ou les muqueuses, et qui ne nécessite généralement pas d'autorisation de mise sur le marché ou AMM.
Composition pharmaceutique : on entend dans la description et dans les revendications par les termes "composition pharmaceutique" une composition qui est reconnue comme médicament et qui fait donc l'objet d'une autorisation de mise sur le marché ou AMM. Dans le cadre de l'invention, cette composition pharmaceutique est particulièrement destinée à être appliquée topiquement sur la peau, le derme ou les muqueuses.
Lipides peroxydés : dans le cadre de la présente description et les revendications, on entend par "lipides peroxydés" des corps gras peroxydés appartenant à la famille des huiles naturelles hyperoxygénées. Cette hyperoxygénation est généralement obtenue par saturation en oxygène et exposition intensive et contrôlée aux ultraviolets de façon à présenter un taux de peroxydation donné. Un tel lipide d'origine végétale se trouve dans les huiles naturelles d'origine végétales, dont la description a lieu plus loin.
Zinc : on entend dans le cadre de la présente description et des revendications par le terme « zinc » un composé dermatologiquement compatible constitué d'oxyde de zinc.

Ainsi, l'invention vise à la fois des compositions dermatologiques et/ou pharmaceutiques pour application topique.

De ce fait , selon un premier aspect, l'invention porte sur de nouvelles compositions, dermatologiques et/ou pharmaceutiques, topiques, contenant, en tant qu'agents actifs ou constituants essentiels, une association de lipides peroxydés et un composé de zinc compatible avec une utilisation dermatologique constitué par de l'oxyde de zinc.

Par "agents actifs ou constituants essentiels" au sens de l'invention, il faut comprendre que les lipides peroxydés et l'oxyde de zinc sont les agents actifs ou constituants principaux des agents actifs de la composition. Dans le cadre d'une composition sous forme de gel huileux leur pourcentage total en poids est généralement d'au moins 50%, en particulier d'au moins 55%, et encore mieux d'au moins 70%. Par ailleurs dans le cadre d'une composition sous forme d'une crème, élaborée à partir d'une émulsion, en particulier de type huile dans eau, la phase huileuse de ladite émulsion comprend de 1 à 25% en poids des lipides peroxydés par rapport à ladite composition.

La présence du lipide peroxydé permettra la formation sur la peau ou la muqueuse à traiter d'un film lipidique qui agira comme un véritable pansement à l'abri duquel la régénération naturelle du tissu se trouve accélérée et, par conséquent, la cicatrisation facilitée.

L'oxyde de zinc permettra à la fois d'améliorer l'effet cicatrisant de la composition et d'améliorer en outre l'adhésion du film sur la peau ou la muqueuse.

Il contribuera en outre, à une meilleure conservation de la composition.

Ces compositions mises au point tout particulièrement pour le traitement des symptômes de l'herpès, en particulier de l'herpès labial ou génital, pourront également être utilisées dans toutes les applications dermatologiques où l'on cherche une amélioration de la cicatrisation ou de la reconstitution d'un épiderme lésé ou une action antalgique sur la peau.

Un avantage tout particulier des compositions de l'invention est que, dans leurs formulations actuellement préférées, elles n'induisent aucun effet pharmacologique, métabolique ou immunologique du fait qu'elles ne contiennent pas de composés pharmacologiques et sont constituées essentiellement de lipides peroxydés, à savoir des triesters de glycérol oxydés ; et de l'oxyde de zinc pour son effet de synergie, améliorer la galénique et protéger la composition, les autres constituants étant pour l'essentiel des arômes ou des parfums, des conservateurs ou des agents destinés à régler la viscosité de la composition pour améliorer l'application topique sur la peau ou les muqueuses visées.

Un avantage particulier de ce type de compositions et du mode d'action mis en oeuvre lors de son application (formation d'un film lipidique sur la zone à traiter) est qu'elles s'avèrent particulièrement intéressantes à une époque où l'on recherche tout particulièrement des modes de traitement aussi peu agressifs que possible pour l'organisme. Ces compositions permettent en outre de satisfaire à la réglementation de la Communauté Européenne concernant les dispositifs médicaux et des organismes américains tels que la FDA (Food and Drug Administration) concernant les dispositifs médicaux (Medical Devices) ou les produits de type « Monographe » (Monograph Products).

Selon un mode de réalisation particulier de l'invention les lipides peroxydés présentent un taux de peroxydation compris entre 5 et 600 milliéquivalents par kg de lipides peroxydés.

Selon un autre mode de réalisation particulier de l'invention, le zinc est présent sous forme d'un composé compatible avec une application dermatologique constitué par de l'oxyde de zinc, ladite composition étant formulée pour une application topique sur la peau ou les muqueuses.

Ainsi, selon encore un mode de réalisation particulier de l'invention, l'invention vise une composition dermatologique et/ou pharmaceutique topique sous forme de gel, comprenant une association, en pourcentage relatifs en poids:
- lipides peroxydés ou TGO de 50 à 99 %
- oxyde de zinc de 1.0 à 25%.

Ainsi, selon encore un autre mode de réalisation particulier de l'invention, l'invention vise une composition dermatologique et/ou pharmaceutique topique sous forme de gel comprenant, en pourcentage relatifs en poids:
- lipides peroxydés ou TGO de 50 à 95 %
- oxyde de zinc de 1.0 à 25%
- un agent gélifiant comprenant du Dioxyde de Silicium de 1 à 10.0%.

Un exemple de dioxyde de Silicium est la silice colloïdale, notamment disponible sur le marché sous le nom de AEROSIL^{®}.

Selon encore un autre mode de réalisation particulier de l'invention, l'invention vise une composition dermatologique et/ou pharmaceutique topique sous forme de gel comprenant, en pourcentage relatifs en poids:
- lipides peroxydés de 50 à 95 % ;
- oxyde de zinc de 1.0 à 25% ;
- un agent gélifiant comprenant du Dioxyde de Silicium de 1 à 10.0% ;
- Eventuellement agent conservateur 0.1 à 1.0
- Eventuellement Arômes 0.5 à 5%

Selon une caractéristique particulière, dans tous les modes de réalisation de l'invention le solde à 100% de la composition de l'invention sera généralement constitué par les huiles peroxydées dans une formulation de gel, ou par l'eau dans une formulation crème.

Comme exemple d'agent conservateur on peut utiliser au moins un paraben tel que le propylparaben.

Outre les agents actifs ou constituants essentiels, c'est-à-dire, l'huile peroxydée et l'oxyde de zinc, et éventuellement l'eau, la composition de l'invention pourra renfermer différents additifs tels que, notamment des parfums ou des arômes choisis en fonction de l'application de la composition, des agents conservateurs ou des agents destinés à régler la viscosité de la composition.

Comme exemple d'arômes, on pourra utiliser tous types d'arômes tels que orange, Pamplemousse, menthe et en particulier la composition pourra comprendre comme arômes, en poids :
- orange-Pamplemousse 2.5%
- Arôme menthe 1.5%

La composition pourra en outre, contenir différents adjuvants de formulation destinés notamment à améliorer la galénique, à faciliter l'application ou à améliorer la protection de la composition.

De tels adjuvants de formulation seront, de façon classique, des agents émulsionnants, émulsifiants, des tensio-actifs, des agents mouillants, émollients, conservateurs ou antioxydants.

Comme exemple d'agent conservateur on peut utiliser au moins un paraben tel que le Propylparaben.

Les lipides peroxydés utilisés pour la préparation des compositions de l'invention résultent de la peroxydation de corps gras insaturés. Le degré de peroxydation est mesuré selon la norme ISO 3960.

Pour la préparation des compositions de la présente invention, on choisira des lipides peroxydés présentant un taux de peroxydation compris entre 5 et 600 milliéquivalents par kg, de préférence entre 30 et 500 milliéquivalents par kg.

Ce taux de peroxydation sera de façon encore plus avantageuse compris entre 50 et 300 milliéquivalents par kg, de préférence encore entre 50 et 150 milliéquivalents par kg.

Les lipides peroxydés préférés utilisés selon l'invention résultent de la peroxydation de lipides ou corps gras d'origine naturelle, de préférence d'origine végétale, de préférence encore de lipides issus d'une huile végétale naturelle.

A titre d'exemples d'huile naturelle choisie selon l'invention, on citera, l'huile d'amande douce, l'huile de noisette, l'huile d'arachide, l'huile de maïs, l'huile de pépin de raisin, l'huile de sésame et l'huile de carthame. On pourra également utiliser un mélange de ces huiles.

Selon une variante particulièrement préférée de l'invention, on choisira l'huile de maïs peroxydée et tout particulièrement une huile de maïs présentant un taux de peroxydation compris entre 5 et 600 milliéquivalents par kg.

Les lipides peroxydés utilisés selon l'invention sont d'une façon avantageuse constitués, à titre de principes actifs ou constituants majoritaires par rapport au total des principes actifs, représentant généralement au moins 80 % en poids , de triglycérides répondant à la formule : 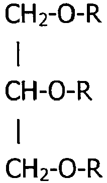 dans laquelle les radicaux R sont majoritairement représentés par des acides insaturés en C18 partiellement peroxydés, en fonction du taux de peroxydation dudit lipide.

Comme exposé précédemment, les compositions de l'invention contiennent du zinc sous forme d'un composé de zinc dermatologiquement acceptable constitué par de l'oxyde de zinc connu notamment pour ses propriétés couvrantes et protectrices facilitant ainsi la cicatrisation et la diminution de la douleur et de l'inflammation.

Selon une variante avantageuse de l'invention, l'oxyde de zinc, se trouvera dans la composition sous une forme micronisée, favorisant ainsi l'application de la composition sur la peau ou la muqueuse concernée.

L'oxyde de zinc se trouvera avantageusement dans la composition à une concentration qui est celle habituellement utilisée dans les applications dermatologiques du zinc.

Ainsi, la composition de l'invention contiendra avantageusement de 0,1 à 25% en poids d'oxyde de zinc, de préférence de 1 à 25% d'oxyde de zinc.

Les compositions de l'invention sont préparées avantageusement sous la forme d'un gel ou d'une crème.

Lorsque la composition sera sous forme d'un gel, il s'agira généralement d'un gel huileux.

Les lipides peroxydés constitueront l'huile du gel huileux où la proportion d'huile peroxydée sera en particulier comprise entre 50 et 95% en poids. Ce gel pourra contenir en outre un agent permettant d'obtenir la viscosité recherchée pour une bonne application topique du gel. Cet agent de viscosité sera de préférence la silice colloïdale, le gel contiendra en outre la proportion voulue d'oxyde de zinc. Un mélange des lipides peroxydés et d'oxyde de zinc est fait préalablement aux opérations de gélification ou de fabrication des émulsions telles que crèmes et pommades.

La composition pourra éventuellement contenir un ou plusieurs conservateurs et/ou un parfum ou un arôme, choisi en fonction du type d'application recherchée.

Bien entendu, le parfum ou l'arôme sera choisi en fonction du type d'application visée.

Par exemple, pour le traitement de l'herpès labial on choisira de préférence un arôme de type arôme alimentaire. On évitera l'utilisation de tels arômes ou parfums dans le cas du traitement de l'herpès génital.

Lorsque la composition est sous forme d'une crème, élaborée à partir d'une émulsion, il s'agit en particulier d'une émulsion de type huile dans eau. Dans une telle émulsion, la phase huileuse sera constituée des lipides peroxydés et représentera avantageusement de 1 à 25 % en masse de ladite composition. Il pourra également contenir de façon générale, tout adjuvant de formulation classiquement utilisé dans le domaine.

Comme exposé précédemment, la composition de l'invention présente l'avantage de répondre à la définition d'un dispositif médical selon la réglementation européenne en vigueur 93/42/CEE.

Ainsi, la composition de l'invention pourra être utilisée en tant que dispositif médical dans de très nombreuses applications où l'on cherche à favoriser notamment la cicatrisation ou la régénération de la peau, ou des muqueuses, notamment par formation d'un film protecteur sur la peau ou les muqueuses.

Ainsi, ce dispositif médical est tout particulièrement destiné à favoriser le renouvellement des cellules du derme et ainsi aider à la cicatrisation et/ou à la reconstitution d'un épiderme lésé et/ou à agir comme agent antalgique sur la peau ou les muqueuses.

Selon un deuxième aspect l'invention couvre aussi l'utilisation d'une association de lipides peroxydés et d'un composé de zinc compatible avec une utilisation dermatologique constitué par de l'oxyde de zinc pour la préparation d'un dispositif médical tel que précédemment défini ou tel que résultant de la description suivante, ledit dispositif médical étant destiné à aider la cicatrisation ou la régénération de la peau ou des muqueuses et/ou le renouvellement des cellules du derme et ainsi aider à la reconstitution d'un épiderme lésé et/ou à agir comme agent antalgique sur la peau ou les muqueuses.

En particulier, ledit dispositif médical est destiné au traitement des symptômes de l'herpès : douleur, brûlure, prurit, picotements, oedème, ulcérations/érosions, érythème, papules, vésicules, croûtes.

Selon une variante, ledit dispositif médical est destiné au traitement des symptômes de brûlures , post radiothérapie ou induits par des traitements pré-cancéreux et cancéreux de la peau, ou toute autre brûlure, notamment les coups de soleil.

Selon une autre variante, ledit dispositif médical est destiné au traitement des symptômes des prurits chroniques sine maria, des démangeaisons et lésions de grattage.

Selon une autre caractéristique particulière, ledit dispositif médical est destiné au traitement de l'herpès labial ou génital.

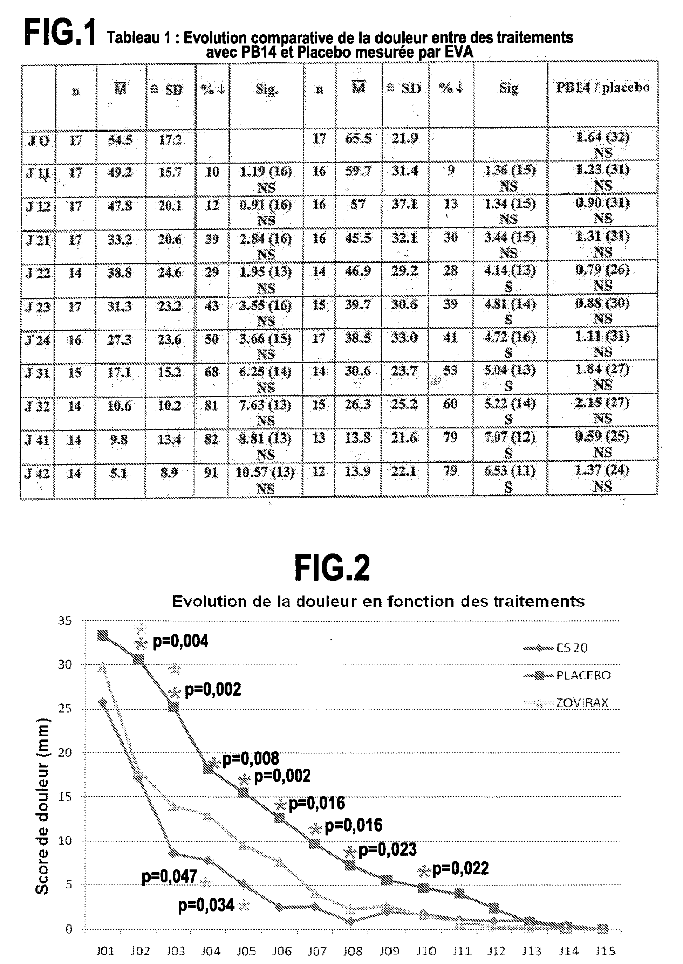

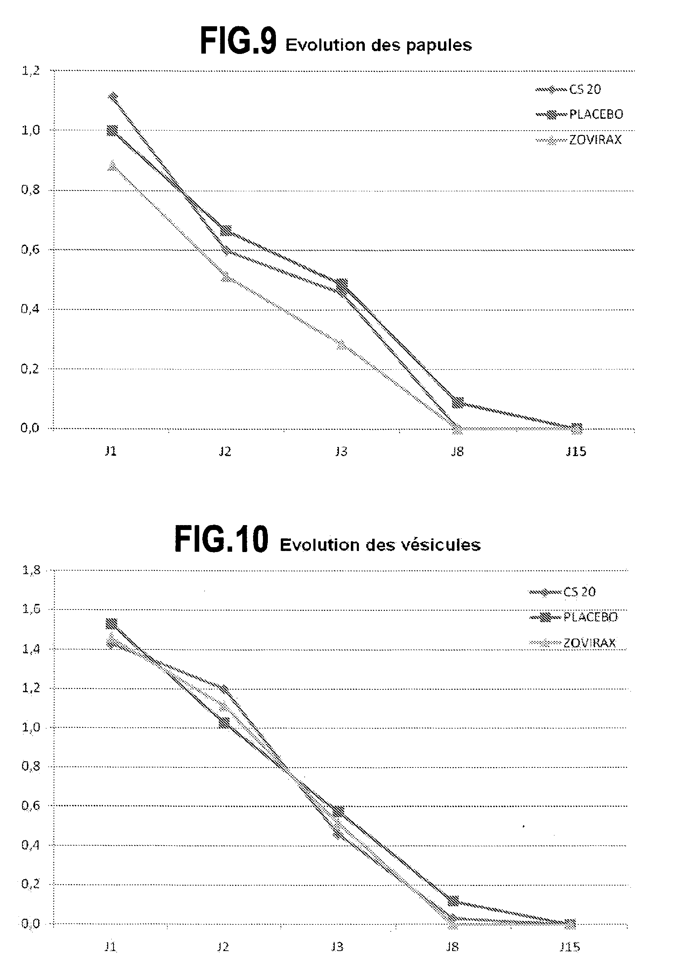

Selon un troisième aspect l'invention couvre encore une méthode de traitement d'au moins une zone de la peau, de l'épiderme ou des muqueuses en ayant besoin avec une composition telle que précédemment définie ou telle que résultant de la description suivante complétée par les dessins, dont les figures 2 à 12 font partie intégrante de l'invention ; et ce notamment dans le but d'aider à la cicatrisation ou la régénération de la peau ou des muqueuses et/ou le renouvellement des cellules du derme et ainsi aider à la reconstitution d'un épiderme lésé et/ou à agir comme agent antalgique sur la peau ou les muqueuses.

Selon une variante de réalisation, cette méthode de traitement sera destinée au traitement au traitement des symptômes de l'herpès : douleur, brûlure, prurit, picotements, oedème, ulcérations/érosions, érythème, papules, vésicules, croûtes ainsi que brûlures domestiques, post radiothérapie ou induites par l'exposition solaire.

Selon une autre variante de réalisation, cette méthode de traitement sera destinée au traitement de l'herpès labial ou génital.

Selon encore une variante de réalisation, cette méthode de traitement comprendra l'application de la composition 3 à 5 fois par jour sur la zone de la peau ou de la muqueuse concernée.

La composition de l'invention, en formant lors de son application un film protecteur sur les différentes lésions de la peau ou des muqueuses concernées trouve son application pour aider à la cicatrisation ou la régénération de la peau ou des muqueuses et/ou le renouvellement des cellules du derme et ainsi aider à la reconstitution d'un épiderme lésé et/ou à agir comme agent antalgique sur la peau ou les muqueuses ; et tout particulièrement dans le traitement au traitement des symptômes de l'herpès : douleur, brûlure, prurit, picotements, oedème, ulcérations/érosions, érythème, papules, vésicules, croûtes ainsi que brûlures domestiques, post radiothérapie ou induites par l'exposition solaire.

Comme cela ressort des exemples, cette composition est tout particulièrement utile dans le traitement des symptômes de l'herpès labial ou génital.

Dans toutes les applications visées, on a pu observer une meilleure adhésion du film formé sur la peau ou la muqueuse lésée du fait de la présencede l'oxyde de zinc.

### DESCRIPTION DES FIGURES

- la figure 1 représente au Tableau 1 l'évolution comparative de la douleur entre les traitements avec le produit comparatif dénommé PB14, conforme à l'exemple 1 ci-après et décrit à la page 5, lignes 5 à 8 de FR-2 591 109B1 antérieur du Déposant; et le placébo mesurée en EVA.
- la figure 2 représente les résultats du test d'évolution de la douleur en fonction des traitements respectivement pour le placebo, courbe avec les carrés ; pour le témoin positif Zovirax^{®}, courbe joignant les triangles, et pour le produit selon l'invention code CS20, courbe avec les losanges, avec les jours de J01 à J15 en abscisse ; et en ordonnées sur une échelle de 0 à 35, le score de la douleur exprimée en millimètres sur la base d'une réglette d'évaluation de la douleur qui est la seule méthode reconnue.
- la figure 3 représente l'évolution du score de brûlure en fonction des traitements de J1 à J15 en abscisse et en fonction du score de brûlure en millimètre sur la même échelle de 0,0 à 35,0, en ordonnées, entre le placebo, courbe joignant les carrés, le témoin positif Zovirax^{®}, courbe joignant les triangles, et le produit selon l'invention code CS20, courbe joignant les losanges ;
- la figure 4 représente la courbe d'évolution de la somme des scores subjectifs en fonction des traitements de J1 à J15 en abscisse et le score somme subjectif en millimètres de 0,0 à 160,0, entre le placebo, le témoin positif Zovirax°, et les produits de l'invention code CS20 ;
- la figure 5 représente la courbe d'évolution du prurit de J1 à J15 en abscisse et fonction du score d'évolution du Prurit en ordonnées, sur une échelle notée de 0,0 à 40,0 entre le même placebo, le même témoin positif Zovirax^{®} et le produit de l'invention code CS20 ;
- la figure 6 représente l'évolution des picotements de J1 à J15 en abscisse, en fonction de la cotation d'évolution des picotements en ordonnées sur une échelle notée de 0,0 à 50,0, entre le même placebo, le même témoin positif Zovirax^{®}, et le produit de l'invention code CS20 ;
- la figure 7 représente la courbe d'évolution de l'érythème de J1 à J15 en abscisse, en fonction de la notation d'évolution de l'érythème sur une échelle notée de 0,01 à.1,8 en ordonnées, pour respectivement le placebo, la courbe joignant les carrés ; et le même positif Zovirax^{®}, courbe joignant les triangles, et le CS20, courbe joignant les losanges ;
- la figure 8 représente la courbe d'évolution de l'oedème, de J1 à J15 en abscisse ; en fonction de la cotation de l'évolution de l'oedème en ordonnées sur une échelle notée de 0,0 à 2,0, pour le même placebo ; le même témoin positif Zovirax^{®} et le produit de l'invention code CS20 ;
- la figure 9 représente la courbe d'évolution des papules de J1 à J15 en abscisse ; en fonction de la cotation de l'évolution des papules sur une échelle notée de 0,01 à 1,2, en ordonnées, pour le même placebo ; le même témoin positif Zovirax^{®} et le produit de l'invention code CS20 ;
- la figure 10 représente la courbe d'évolution des vésicules de J1 à J15 en abscisse ; en fonction de la cotation de l'évolution des vésicules en ordonnées, sur une échelle de 0,01 à 1.8, pour le même placebo ; le même témoin positif Zovirax^{®} et le produit de l'invention code CS20 ;
- la figure 11 représente la courbe d'évolution des ulcérations/érosions de J1 à J15 en abscisse ; en fonction de la cotation de l'évolution des ulcérations/érosions sur une échelle de 0,01 à 1.4, respectivement pour le même placebo ; le même témoin positif Zovirax^{®} et le produit de l'invention code CS20 ; et
- la figure 12 représente la courbe d'évolution des croûtes, de J1 à J15 en abscisse ; en fonction de la cotation de l'évolution des croûtes notées de 0,01 à 1.8, en ordonnées, pour le même placebo ; le même témoin positif Zovirax^{®} et le produit de l'invention code CS20.

### EXEMPLES

Dans les exemples qui suivent, les proportions données sont exprimées en pourcentage en poids, la température en °C et la pression est la pression atmosphérique. Par ailleurs, le solde à 100% est toujours constitué par les lipides peroxydés ou par l'eau en cas de forme galénique de crème.

### EXEMPLE 1- COMPOSITION COMPARATIVE code PB 14 selon FR 2.591.109

On prépare une composition comparative conforme à l'exemple du brevet précédent du déposant FR-2 591 109B1, page 5, lignes 5 à 8, à savoir un gel ayant la composition suivante :
- huile de carthame hyperoxygénée 90,7 %
- dioxyde de silicium sous forme de silice micronisée commercialisée sous la marque Aerosil 300^{®} de la société Degussa 6,8 %
- parfum : 2,5 %.

Cette composition porte le nom de code PB 14.

L'huile de carthame peroxydée présente un taux de peroxydation compris entre 30 et 300 exprimés en milliéquivalents par Kg.

Les exemples ci-après utilisent des huiles de maïs peroxydées présentant un taux de peroxydation compris entre 50 et 600 exprimés en milliéquivalents par kg.

### Exemple 2

### Composition destinée au traitement de l'herpès labial

Cette composition est préparée avec les ingrédients suivants, de la manière habituelle, et est codée **CS 20 :**

| Ingrédients, code **CS 20** | % |
|---|---|
| Huile de Maïs Peroxydée | 87,8 % |
| Oxyde de Zinc micronisé | 1,0 % |
| Agent gélifiant Dioxyde de silicium* | 7,0 % |
| Arômes "Orange-Pamplemousse" | 2,5 % |
| Arôme "Menthe" | 1,5 % |
| Agent conservateur Propylparaben | 0,2 % |

| | |
|---|---|
| * silice colloidale Aerosil 300® de DEGUSSA | |

### Exemple 3

### Composition destinée au traitement de l'herpès génital

Cette composition est préparée avec les ingrédients suivants, de la manière habituelle, et est codée CS 21 :

| Ingrédients code CS 21 | % |
|---|---|
| Huile de Maïs Peroxydée | 91,8 % |
| Oxyde de Zinc micronisé | 1,0 % |
| Agent gélifiant, Dioxyde de silicium* | 7,0 % |
| Agent conservateur, Propylparaben | 0,2 % |

| | |
|---|---|
| * silice colloidale Aerosil 300® de DEGUSSA | |

### Exemple 4

### TESTS D'EFFICACITE

### 4-1 TEST D'EFFICACITE DE LA COMPOSITION COMPARATIVE PB14 selon l'EXEMPLE 1

Les tests ont été réalisés de la manière suivante :
Cinq applications par jour pendant treize jours pour de la composition comparative de l'exemple 1, code PB14.

2 groupes de 20 patients par groupe, caractères d'inclusion patients des 2 sexes âgés de plus de 15 ans présentant 2 herpès récurrents avec au minimum 2 poussées dans l'année précédente à la même localisation.
Caractères de non inclusion :
- primo-infection
- des signes cliniques douteux. patients sous traitement anti- viraux ;
- diabète, grossesse
- patients immuno déprimés.

Placébo et un groupe produit à tester.

Les résultats de l'évolution comparative de la douleur, mesurée en EVA, entre les traitements avec le produit comparatif dénommé PB14, conforme à l'exemple 1, décrit ci-dessus, et à la page 5, lignes 5 à 8 de FR-2 591 109B1 antérieur du Déposant; et le placébo, font l'objet du Tableau 1 donné en Figure 1.

La méthode « EVA » est une méthode d'auto évaluation de la douleur, internationalement reconnue par le Praticien, par rapport à des points extrêmes : douleur intolérable et pas de douleur du tout. Le patient, sur une droite de 10 cm, place sa note entre ces 2 extrêmes.

Il peut être observé à partir du Tableau 1 qu'avec les huiles peroxydées du PB 14 utilisées seules, les résultats des études cliniques portant sur la sédation de la douleur des lésions herpétiques ont été décevants, sans pouvoir établir de différence significative entre PB14 et placebo de J0 à J42.

### 4-2 TESTS D'EFFICACITE DES COMPOSITION SELON L'INVENTION CS 20 et CS 21 selon les EXEMPLES 2 et 3

L'élaboration du produit CS20, d'une composition différente du produit antérieur PB14 , justifiait une recherche clinique dont les résultats devaient correspondre à l'attente des malades sur les signes fonctionnels et cliniques de l'herpès notamment vis-à-vis du produit majeur mondialement reconnu à effet anti-viral comprenant l'Acyclovir (ZOVIRAX®) dont l'action revendiquée est de raccourcir la durée de la poussée herpétique, utilisé comme témoin positif.

Il est parfaitement reconnu que le soulagement de la douleur et de la brûlure qui accompagnent la poussée herpétique sont les critères majeurs de qualification de l'efficacité.

L'objet de l'invention était donc de trouver un produit dont l'action bénéfique serait essentiellement orientée dans ce sens. C'est pourquoi les travaux cliniques référencés dans ce texte correspondent à un protocole dont l'apaisement de la douleur et de la brûlure sont les critères principaux. Les signes cliniques accompagnant l'épisode herpétique, principalement oedème, ulcération, érosion, papules, vésicules, croûtes ont aussi été pris en compte.

Le protocole des tests thérapeutiques sur la lésion herpétique recommande cinq applications par jour pendant quinze jours pour la composition de l'invention soit de l'exemple 2, code CS 20, pour l'HERPES LABIAL, soit de l'exemple 3, code CS 21 pour l'HERPES GENITAL ; ainsi que pour le placébo et pour le médicament comprenant l'acyclovir constitué par le ZOVIRAX®.

### POUR L'HERPES LABIAL, composition de l'EXEMPLE 2, CS 20 :

3 groupes de 35 patients par groupe, caractères d'inclusion patients des 2 sexes agés de plus de 18 ans présentant des herpès récurrents avec au minimum 4 poussées dans l'année précédente à la même localisation. Caractères de non inclusion :
- primo-infection
- des signes cliniques douteux.
- patients sous traitement anti- viraux ;
- diabète, grossesse
- patients immuno déprimés.

### POUR L'HERPES GENITAL, composition de l'EXEMPLE 3, CS 21:

3 groupes de 20 patients par groupe, caractères d'inclusion patients des 2 sexes âgés de plus de 18 ans présentant des herpès récurrents avec au minimum 4 poussées dans l'année précédente à la même localisation dans la sphère génitale de l'homme et de la femme.

Pour Chaque TEST :
- un groupe reçoit le placébo ;

Un groupe reçoit le témoin positif Zovirax® ; et
un Groupe reçoit le produit de l'invention code CS 20 et CS21

Caractères de non inclusion :
- primo-infection
- des signes cliniques douteux.
- patients sous traitement anti- viraux ;
- diabète, grossesse
- patients immuno déprimés.

Les résultats donnés aux figures 2 à 12 sont des moyennes statistiques de chaque groupe avec un taux de significativité inférieure à 0.05 pour la douleur comme pour la brûlure, démontrant la supériorité inattendue de la composition de l'invention par rapport non seulement au placébo mais aussi au témoin positif constitué par le médicament anti-viral prescrit majoritairement contre l'Herpès, l'Acyclovir à 5% sous forme crème (ZOVIRAX®).

Dans les figures 2 à 12, la mention d'une étoile pour un point particulier caractérise un point de différence significative pour le produit de l'invention CS20 par rapport au Zovirax® et/ou le Placébo.

Ainsi, la figure 2 montre une différence significative sur la douleur du produit de l'invention CS20 par rapport au placébo de J2 à J12 ; et par rapport au Zovirax® à J4 et J5.

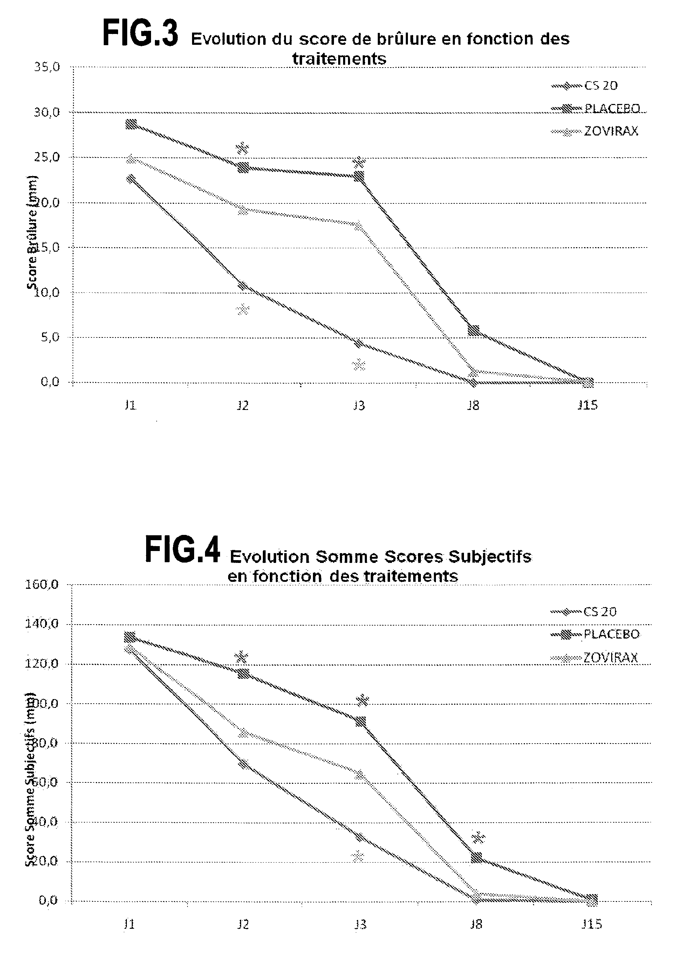

La figure 3 montre une différence significative sur la brûlure du produit de l'invention CS20 par rapport au placébo à J2 avec une valeur de significativité p de 0.004, et à J3 avec une valeur de significativité p de 0.000; et par rapport au Zovirax® à J2 avec une valeur de significativité p de 0.033, et à J3 avec une valeur de significativité p de 0.005.

La figure 4 montre une différence significative sur la somme des scores subjectifs en fonction des traitements du produit de l'invention CS20 par rapport au placébo à J2 avec une valeur de significativité p de 0.008, et à J3 avec une valeur de significativité p de 0.000; et par rapport au Zovirax® à J3 avec une valeur de significativité p de 0.033.

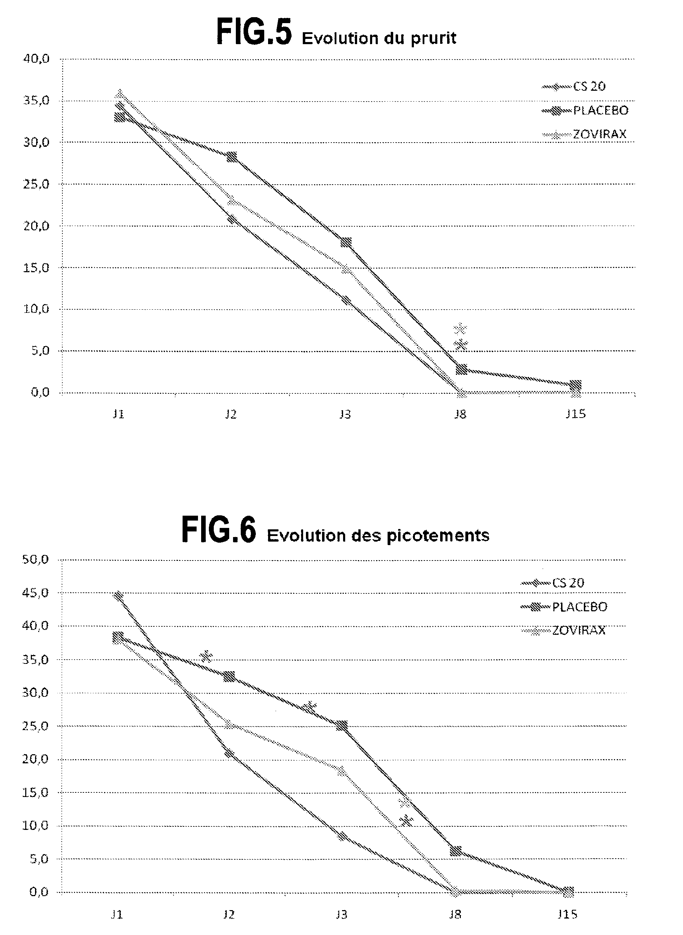

La figure 5 montre l'évolution du prurit où le produit de l'invention CS20 et le Zovirax® sont significatifs par rapport au placébo à J8 .

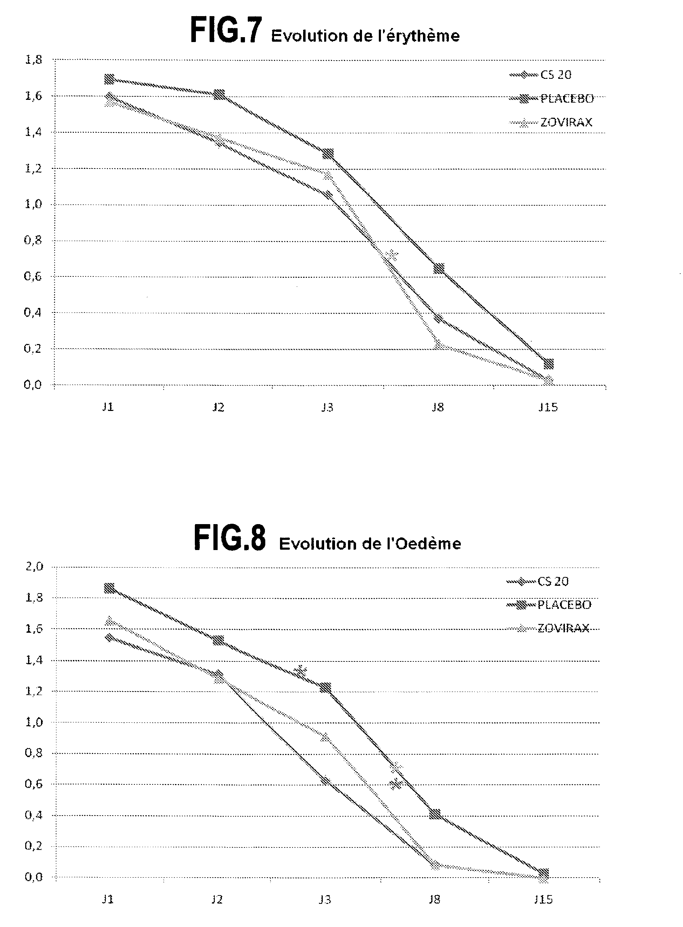

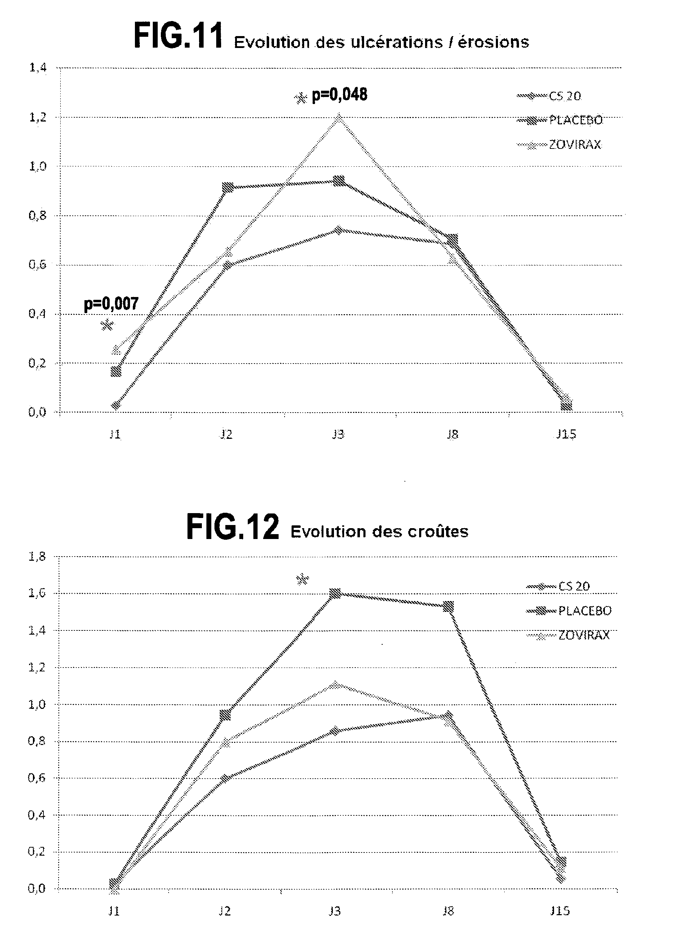

Comme il ressort encore des figures 2 à 12, en sus des signes fonctionnels, l'étude a mis en évidence des résultats significatifs avec la composition de l'invention de l'exemple 2, produit CS20 par rapport au moins le Placébo, pour des signes cliniques comprenant :
- évolution du prurit à J8 , Figure 5;
- évolution des picotements à J2, J3, J8, Figure 6 ;
- évolution de l'érythème vers J8, Figure 7 ;
- évolution de l'oedème à J3 et jusqu'à J8 , Figure 8;
- évolution de l'ulcération/érosion par rapport au ZOVIRAX® à J1 avec une valeur de significativité p inférieure à 0.007 et à J3 avec un p inférieur à 0.048, Figure 11; et
- évolution des croûtes avec une valeur de significativité, par rapport au Placébo à J3, p inférieure à 0.009.

Comme il ressort par ailleurs du Tableau 2 ci-après, le produit de l'invention CS20 est hautement significatif sur la sédation de la douleur en comparaison au placebo et aussi au témoin positif constitué par le médicament anti-viral prescrit majoritairement contre l'Herpès, l'Acyclovir à 5% sous forme crème (ZOVIRAX®).

**TABLEAU 2**

| ANALYSE DE LA QUESTION POSEE EN AUTO QUESTIONNAIRE A J15 A CHAQUE PERSONNE DE TOUS LES GROUPES TESTES | |
|---|---|
| Rappel de la question : | |
| | |
| Par rapport à votre vécu des lésions d'herpès | Choix de réponses |
| **Q1 réduction** : Diriez-vous que le traitement a réduit les sensations douloureuse de l'herpès ? | 2 = tout à fait d'accord |
| | 1 = assez d'accord |
| | 0 = sans position |
| | -1 = pas tellement d'accord |
| | -2 = pas d'accord du tout |

La distribution des réponses est donnée dans le tableau suivant :

| | **CS 20** | PLACEBO | ZOVIRAX | TOTAL |
|---|---|---|---|---|
| -2 | **0** | 6 | 1 | 7 |
| -1 | **3** | 5 | 4 | 12 |
| 0 | **0** | 2 | 0 | 2 |
| 1 | **3** | 11 | 16 | 30 |
| 2 | **29** | 10 | 14 | 53 |
| Total | **35** | 34 | 35 | 104 |

La projection statistique de ces résultats est la suivante :

| | **CS20 vs PLACEBO** | **ZOVIRAX vs Placebo** | **CS20 vs ZOVIRAX** |
|---|---|---|---|
| **Q1 réduction** | P<0.000 pour CS20 | P=0.123 (ns) | P=0.002 pour CS20 |

En conclusion, la composition de l'invention de l'exemple 2, CS20, est très significativement meilleur que le placébo et même que le témoin positif constitué par le médicament anti-viral prescrit majoritairement contre l'Herpès, l'Acyclovir à 5% sous forme crème (ZOVIRAX®) taux de significativité de 0.007.

Des résultats similaires sont attendus pour l'HERPES GENITAL avec la composition de l'exemple 3, CS21.

Au vu de ces résultats, l'invention est applicable au traitement de la peau ou des muqueuses pour des indications de même nature, comprenant : traitement des symptômes de l'herpès : douleur, brûlure, prurit, picotements, oedème, ulcérations/érosions, érythème, papules, vésicules, croûtes ainsi que brûlures domestiques, post radiothérapie ou induites par l'exposition solaire.

### Exemple 5

### Composition dermatologique et/ou pharmaceutique destinée au traitement de l'herpès labial

| Ingrédients | % |
|---|---|
| Huile de Maïs Peroxydée | 87,8 % |
| Oxyde de Zinc micronisé | 1,0 % |
| Agent gélifiant Dioxyde de silicium* | 7,0 % |
| Arôme "Orange-Pamplemousse" | 2,5 % |
| Arôme "Menthe" | 1,5 % |
| Agent conservateur, Propylparaben | 0,2 % |

| | |
|---|---|
| * silice colloidale Aerosil 300® de DEGUSSA | |

Cette composition est appliquée 3 à 5 fois par jour en massages légers sur la zone concernée pendant 6 jours consécutifs à partir de l'apparition des premiers symptômes douloureux. On a constaté une cessation rapide de la douleur et disparition du bouton d'herpès après 3 à 4 jours de traitement. La crise herpétique a été atténuée et sa durée sensiblement diminuée.

Ce test a été réalisé sur 35 volontaires

### Exemple 6

### Composition dermatologique et/ou pharmaceutique destinée au traitement de l'herpès génital.

La composition contient :

| Ingrédients | % |
|---|---|
| Huile de Maïs Peroxydée | 91,8 % |
| Oxyde de Zinc micronisé , | 1,0 % |
| Agent gélifiant, Dioxyde de silicium* | 7,0 % |
| Agent conservateur, Propylparaben | 0,2 % |

| | |
|---|---|
| * silice colloidale | |

Cette composition est appliquée 3 à 5 fois par jour en massages légers sur la zone concernée pendant 8 jours consécutifs à partir de l'apparition des premiers symptômes douloureux.

Ce test est réalisé sur 20 volontaires.

### Exemple 7

### Crème dermatologique à base de lipides peroxydés et d'oxyde de zinc pour le traitement des brûlures, du prurit et des démangeaisons

On peut formuler une crème dermatologique à base de lipides peroxydés et d'oxyde de zinc pour le traitement des brûlures, du prurit et des démangeaisons ayant la composition suivante :

| Ingrédients | % |
|---|---|
| Huile de Maïs Peroxydée | 20,0 % |
| Oxyde de Zinc | 4,0 % |
| Excipient Salcare®* | 2,00 % |
| Agent conservateur, paraben | 0,5 % |
| Excipient Kathon CG®** | 0,05 % |
| Eau déminéralisée | Q.S.P 100 % |

| | |
|---|---|
| Salcare* est un agent cosmétique de chez CIBA permettant un contrôle de viscosité et induisant une couleur blanche. | |

Kathon CG®** est un agent cosmétique de chez SEPPIC permettant aussi un contrôle de viscosité et induisant une couleur blanche.

Cette crème pourra être appliquée 2 à 3 fois par jour pendant 10 jours sur des lésions de grattage avec démangeaisons ou prurit, et sur des brûlures domestiques, post radiothérapie, ou dues à une exposition solaire.

## Revendications

1. Composition dermatologique et/ou pharmaceutique, **caractérisée en ce qu'**elle contient, à titre d'agents actifs essentiels, une association de lipides peroxydés et du zinc sous forme d'un composé compatible avec une utilisation dermatologique constitué par de l'oxyde de zinc, ladite composition étant formulée pour une application topique sur la peau ou les muqueuses.

2. Composition selon la revendication 1, **caractérisée en ce que** les lipides peroxydés présentent un taux de peroxydation compris entre 5 et 600 milliéquivalents par kg de lipides peroxydés.

3. Composition selon la revendication 1 ou 2, **caractérisée en ce que** lesdits lipides peroxydés présentent un taux de peroxydation compris entre 30 et 500 milliéquivalents par kg, de préférence entre 50 et 300 milliéquivalents par kg, de préférence encore entre 50 et 150 milliéquivalents par kg de lipides peroxydés.

4. Composition selon l'une des revendications 1 à 3, **caractérisée en ce que** lesdits lipides peroxydés comprennent, à titre d'agents actifs ou constituants majoritaires par rapport au total des principes actifs, des triglycérides partiellement oxydés répondant à la formule générale : 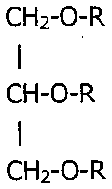 dans laquelle les radicaux R sont des acides insaturés en C18 partiellement peroxydés.

5. Composition selon l'une des revendications 1 à 4, **caractérisée en ce que** lesdits lipides peroxydés sont obtenus par peroxydation de lipides ou de corps gras d'origine naturelle, de préférence de lipides issus d'une huile végétale naturelle.

6. Composition selon la revendication 5, **caractérisée en ce que** l'huile naturelle est choisie dans le groupe constitué de l'huile d'amande douce, de l'huile de noisette, de l'huile d'arachide, de l'huile de maïs, de l'huile de pépin de raisin, de l'huile de sésame et de l'huile de carthame et de leurs mélanges.

7. Composition selon l'une des revendications 1 à 6, **caractérisée en ce que** l'oxyde de zinc est sous une forme micronisée.

8. Composition selon l'une des revendications 1 à 7, **caractérisée en ce qu'**elle contient de 0,1 à 25% en poids d'oxyde de zinc.

9. Composition selon l'une des revendications 1 à 8, **caractérisée en ce qu'**elle contient de 1 à 25 % d'oxyde de zinc.

10. Composition selon l'une des revendications 1 à 9, **caractérisée en ce qu'**elle est sous la forme d'un gel huileux ou d'une crème.

11. Composition selon la revendication 10, **caractérisée en ce que** ledit gel huileux est constitué de lipides peroxydés, d'oxyde de zinc, d'un agent gélifiant, en particulier de silice colloïdale et éventuellement de conservateurs et/ou de parfum ou d'arôme et/ ou d'adjuvants de formulation destinés à améliorer la galénique ou faciliter l'application de la composition.

12. Composition selon l'une des revendications 1 à 11, **caractérisée en ce qu'**elle se présente sous une forme de gel comprenant une association, en pourcentage en poids:
- Lipides peroxydés de 50 à 99 %
- Oxyde de Zinc micronisé de 1.0 à 25%.

13. Composition selon l'une des revendications 1 à 12, **caractérisée en ce qu'**elle se présente sous une forme de gel comprenant, en pourcentage relatifs en poids:
- lipides peroxydés de 50 à 95 % ;
- oxyde de zinc de 1.0 à 25% ;
- un agent gélifiant comprenant du Dioxyde de Silicium de 1 à 10.0% ;
- Eventuellement agent conservateur 0.1 à 1.0
- Eventuellement Arômes 0.5 à 5%

14. Composition selon l'une des revendications 1 à 10, **caractérisée en ce qu'**elle est sous la forme d'une crème, élaborée à partir d'une émulsion, en particulier de type huile dans eau, la phase huileuse de ladite émulsion étant constituée des lipides peroxydés représentant de 1 à 25% en poids de ladite composition.

15. Dispositif médical, **caractérisé en ce qu'**il est constitué d'une composition selon l'une des revendications 1 à 14.

16. Utilisation d'une composition selon les revendications 1-14, en tant que dispositif médicale selon la revendication 15 pour favoriser la cicatrisation ou la régénération de la peau ou des muqueuses, et/ou le renouvellement des cellules du derme et ainsi aider à la reconstitution d'un épiderme lésé et/ou à agir comme agent antalgique sur la peau ou les muqueuses.

17. Utilisation selon la revendication 16, **caractérisée en ce que** ladite composition en tant que dispositif médical est destinée au traitement des symptômes de l'herpès : douleur, brûlure, prurit, picotements, oedème, ulcérations/érosions, érythème, papules, vésicules ou croûtes.

18. Utilisation selon la revendication 16, **caractérisée en ce que** ladite composition en tant que dispositif médical est destinée au traitement des symptômes de brûlures, post radiothérapie ou induits par des traitements pré-cancéreux et cancéreux de la peau, ou toute autre brûlure, notamment les coups de soleil.

19. Utilisation selon la revendication 16, **caractérisée en ce que** ladite composition en tant que dispositif médical est destinée au traitement des symptômes des prurits chroniques sine materia, des démangeaisons et lésions de grattage.

20. Utilisation selon la revendication 16 ou 17, **caractérisé en ce que** ladite composition en tant que dispositif médical est destinée au traitement de l'herpès labial ou génital.

## Patentansprüche

1. Dermatologische und/oder pharmazeutische Zusammensetzung, **dadurch gekennzeichnet, dass** sie als essentielle Wirkstoffe eine Kombination aus peroxidierten Lipiden und dem Zink in Form einer mit einer dermatologischen Verwendung vereinbaren Verbindung, die von Zinkoxid gebildet ist, enthält, wobei die Zusammensetzung für eine topische Anwendung auf der Haut oder den Schleimhäuten formuliert ist.

2. Zusammensetzung nach Anspruch 1, **dadurch gekennzeichnet, dass** die peroxidierten Lipide einen Peroxidationsgrad im Bereich zwischen 5 und 600 Milliäquivalenten pro kg peroxidierter Lipide aufweisen.

3. Zusammensetzung nach Anspruch 1 oder 2, **dadurch gekennzeichnet, dass** die peroxidierten Lipide einen Peroxidationsgrad im Bereich zwischen 30 und 500 Milliäquivalenten pro kg, vorzugsweise zwischen 50 und 300 Milliäquivalenten pro kg, weiterhin vorzugsweise zwischen 50 und 150 Milliäquivalenten pro kg peroxidierter Lipide aufweisen.

4. Zusammensetzung nach einem der Ansprüche 1 bis 3, **dadurch gekennzeichnet, dass** die peroxidierten Lipide als Wirkstoffe oder Hauptbestandteile bezogen auf die Summe der Wirkstoffe, teilweise oxidierte Triglyceride umfassen, die der allgemeinen Formel: 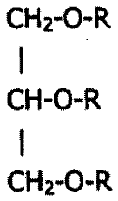 entsprechen, worin die Reste R teilweise peroxidierte ungesättigte C18-Säuren sind.

5. Zusammensetzung nach einem der Ansprüche 1 bis 4, **dadurch gekennzeichnet, dass** die peroxidierten Lipide durch Peroxidation von Lipiden oder von Fetten natürlicher Herkunft, vorzugsweise von Lipiden aus einem natürlichen Pflanzenöl, erhalten werden.

6. Zusammensetzung nach Anspruch 5, **dadurch gekennzeichnet, dass** das natürliche Öl aus der Gruppe bestehend aus Süßmandelöl, Haselnussöl, Erdnussöl, Maisöl, Traubenkernöl, Sesamöl und Safloröl und deren Mischungen ausgewählt ist.

7. Zusammensetzung nach einem der Ansprüche 1 bis 6, **dadurch gekennzeichnet, dass** das Zinkoxid in einer mikronisierten Form vorliegt.

8. Zusammensetzung nach einem der Ansprüche 1 bis 7, **dadurch gekennzeichnet, dass** sie 0,1 bis 25 Gew.-% Zinkoxid enthält.

9. Zusammensetzung nach einem der Ansprüche 1 bis 8, **dadurch gekennzeichnet, dass** sie 1 bis 25 % Zinkoxid enthält.

10. Zusammensetzung nach einem der Ansprüche 1 bis 9, **dadurch gekennzeichnet, dass** sie in Form eines öligen Gels oder einer Creme vorliegt.

11. Zusammensetzung nach Anspruch 10, **dadurch gekennzeichnet, dass** das ölige Gel aus peroxidierten Lipiden, Zinkoxid, einem Geliermittel, insbesondere kolloidalem Siliciumdioxid, und eventuell Konservierungsmitteln und/oder Parfum oder Aroma und/oder Formulierungszusätzen, die dazu bestimmt sind, die Galenik zu verbessern oder das Auftragen der Zusammensetzung zu erleichtern, besteht.

12. Zusammensetzung nach einem der Ansprüche 1 bis 11, **dadurch gekennzeichnet, dass** sie in einer Gelform vorliegt, die eine Kombination, in Gewichtsprozent, umfasst:
- peroxidierte Lipide, 50 bis 99 %
- mikronisiertes Zinkoxid, 1,0 bis 25 %.

13. Zusammensetzung nach einem der Ansprüche 1 bis 12, **dadurch gekennzeichnet, dass** sie in einer Gelform vorliegt, die in Gewichtsrelativprozent umfasst:
- peroxidierte Lipide, 50 bis 95 %;
- Zinkoxid, 1,0 bis 25 %;
- ein Geliermittel, das Siliciumdioxid umfasst, 1 bis 10,0 %;
- eventuell Konservierungsmittel, 0,1 bis 1,0;
- eventuell Aromen, 0,5 bis 5 %.

14. Zusammensetzung nach einem der Ansprüche 1 bis 10, **dadurch gekennzeichnet, dass** sie in Form einer Creme vorliegt, die aus einer Emulsion, insbesondere vom Typ Öl-in-Wasser hergestellt ist, wobei die ölige Phase der Emulsion von den peroxidierten Lipiden gebildet ist, die 1 bis 25 Gew.-% der Zusammensetzung ausmachen.

15. Medizinprodukt, **dadurch gekennzeichnet, dass** es von einer Zusammensetzung nach einem der Ansprüche 1 bis 14 gebildet ist.

16. Verwendung einer Zusammensetzung nach den Ansprüchen 1 bis 14, als Medizinprodukt nach Anspruch 15, um die Wundheilung oder die Regeneration der Haut oder der Schleimhäute und/oder die Erneuerung der Zellen der Dermis zu fördern und somit zur Wiederherstellung einer verletzten Epidermis und/oder zur Wirkung als schmerzstillendes Mittel auf der Haut oder den Schleimhäuten beizutragen.

17. Verwendung nach Anspruch 16, **dadurch gekennzeichnet, dass** die Zusammensetzung als Medizinprodukt für die Behandlung der Symptome von Herpes: Schmerz, Brennen, Juckreiz, Prickeln, Ödem, Geschwürbildungen/Erosionen, Erythem, Quaddeln, Bläschen oder Schorf, bestimmt ist.

18. Verwendung nach Anspruch 16, **dadurch gekennzeichnet, dass** die Zusammensetzung als Medizinprodukt für die Behandlung der Symptome von Verbrennungen, nach Strahlentherapie oder durch Behandlungen von Hautkrebsvorstufen und Hautkrebs induziert, oder jeder anderen Verbrennung, insbesondere Sonnenbränden bestimmt ist.

19. Verwendung nach Anspruch 16, **dadurch gekennzeichnet, dass** die Zusammensetzung als Medizinprodukt für die Behandlung der Symptome von chronischem Pruritus sine materia, Hautjucken und Kratzverletzungen bestimmt ist.

20. Verwendung nach Anspruch 16 oder 17, **dadurch gekennzeichnet, dass** die Zusammensetzung als Medizinprodukt für die Behandlung von Lippen- oder Genitalherpes bestimmt ist.

## Claims

1. A dermatological and/or pharmaceutical composition, **characterized in that** it contains, as essential active agents, an association of peroxidized lipids and of zinc in mineral form or in the form of a compound compatible with dermatological use constituted of zinc oxide, said composition being formulated for topical application on the skin or the mucous membranes.

2. A composition according to claim 1, **characterized in that** the peroxidized lipids present a peroxidation level lying in the range 5 to 600 milliequivalents per kg of peroxidized lipids.

3. A composition according to claim 1 or claim 2, **characterized in that** the peroxidized lipids present a peroxidation level lying in the range 30 to 500 milliequivalents per kg, preferably in the range 50 to 300 milliequivalents per kg, more preferably in the range 50 to 150 milliequivalents per kg of peroxidized lipids.

4. A composition according to any one of claims 1 to 3, **characterized in that** said peroxidized lipids comprise, at active agents or as majority constituents relative to the total of active principles, partially oxidized triglycerides satisfying the general formula: 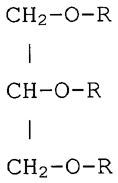 in which the radicals R are partially peroxidized C₁₈ unsaturated acids.

5. A composition according to any one of claims 1 to 4, **characterized in that** said peroxidized lipids are obtained by peroxidizing lipids or fats of natural origin, preferably lipids derived from natural vegetable oil.

6. A composition according to claim 5, **characterized in that** the natural oil is selected from the group constituted by sweet almond oil, hazelnut oil, peanut oil, corn oil, grape seed oil, sesame oil, and safflower oil, and mixtures thereof.

7. A composition according to any one of claims 1 to 6, **characterized in that** the zinc oxide is in a micronized form.

8. A composition according to any one of claims 1 to 7, **characterized in that** it contains 0.1% to 25% by weight of zinc oxide.

9. A composition according to any one of claims 1 to 8, **characterized in that** it contains 1% to 25% by weight of zinc oxide.

10. A composition according to any one of claims 1 to 9, **characterized in that** it is in the form of an oily gel or a cream.

11. A composition according to claim 10, **characterized in that** said oily gel is constituted by peroxidized lipids, zinc oxide, a gelling agent, in particular colloidal silica and optionally preservatives and/or fragrance or flavoring and/or formulation additives for improving the dosage form or for facilitating application of the composition.

12. A composition according to any one of claims 1 to 11, **characterized in that** it is in the form of a gel comprising an association in percentages by weight of:
· peroxidized lipids from 50% to 99; and
· micronized zinc oxide from 1.0% to 25%.

13. A composition according to any one of claims 1 to 12, **characterized in that** it is in the form of a gel comprising, in relative percentages by weight:
· peroxidized lipids from 50% to 95%;
· zinc oxide from 1.0% to 25%;
· a gelling agent comprising silicon dioxide from 1% to 10.0%;
. optionally a preservative agent from 0.1% to 1.0%; and
· optionally flavoring from 0.5% to 5%.

14. A composition according to any one of claims 1 to 10, **characterized in that** it is in the form of a cream, prepared from an emulsion, in particular of the oil-in-water type, the oily phase of said emulsion being constituted by peroxidized lipids representing 1% to 25% by weight of said composition.

15. A medical device, **characterized in that** it is constituted by a composition according to any one of claims 1 to 14.

16. The use of a composition according to claims 1 to 14, as a medical device according to claim 15 for promoting healing or regeneration of skin or mucous membranes and/or renewal of dermis cells, as well as for helping in reconstitution of damaged epidermis and/or acting as an analgesic agent on the skin or the mucous membranes.

17. The use according to claim 16, **characterized in that** said composition as medical device is for treating symptoms of herpes: pain, burning, itching, pricking, edema, ulceration/erosion, erythema, papules, vesicles or crusts.

18. The use according to claim 16, **characterized in that** said composition as medical device is for treatment of symptoms of burns post radiotherapy, or induced by treatment of pre-cancerous or cancerous of skin , or any other burn, notably sun burns.

19. The use according to claim 16, **characterized in that** said composition as medical device is for treatment of sine materia chronic pruritus, itching, lesions caused by scratching.

20. The use according to claim 16 or claim 17, **characterized in that** said composition as medical device is for the treatment of labial or genital herpes.
